# EUROPEAN PATENT SPECIFICATION

(11) **EP 2 070 427 B1**
(45) Date of publication and mention of the grant of the patent: **26.09.2012**
(21) Application number: 08020212.0
(22) Date of filing: 20.11.2008
(51) Int. Cl.: A23K 1/16, A23K 1/18

(54) **Use of essential oil compounds as histomonastat**
Verwendung von essentiellen Ölzusammensetzungen als Histomonastat
Utilisation de composés d'huile essentielle en tant qu'histomonastat

(30) Priority: 11.12.2007 EP 07023954
(43) Date of publication of application: 17.06.2009
(73) Proprietor: DSM IP Assets B.V., 6411 TE Heerlen (NL)
(72) Inventor: Frehner, Marco, 1185 Mont-sur-Rolle (CH); Losa, Riccardo, 1145 Biére (CH); Archain, Didier, 92500 Rueil Malmaison (FR)
(74) Representative: Schwander, Kuno

(56) References cited:
- EP-A- 1 266 578
- WO-A-2004/089108
- WO-A-2004/091307
- WO-A2-2008/003007
- CA-A- 735 458
- CN-A- 1 303 610
- JP-A- 5 176 689
- US-A1- 2008 032 021
- HAFEZ MOHAMED HAFEZ & RÜDIGER HAUCK: "Efficacy of a herbal product against Histomonas meleagridis after experimental infection of turkey poults" 19000101, vol. 5, no. 60, 9 June 2006 (2006-06-09), pages 436-442, XP008101863
- ZENNER L ET AL: "IN VITRO EFFECT OF ESSENTIAL OILS FROM CINNAMOMUM AROMATICUM, CITRUS LIMON AND ALLIUM SATIVUM ON TWO INTESTINAL FLAGELLATES OF POULTRY, TETRATRICHOMONAS GALLINARUM AND HISTOMONAS MELEAGRIDIS" PARASITE, PRINCEPS EDITIONS, FR, vol. 10, no. 2, 1 January 2003 (2003-01-01), pages 153-157, XP008034124 ISSN: 1252-607X
- UTIYAMA C E ET AL: "Effects of antimicrobials, prebiotics, probiotics and herbal extracts on intestinal micobiology, diarrhea incidence and performance of weanling pigs /Efeitos de antimicroblanos, prebioticos e extratos vegetais sobre a microbiotica intestinal,a frequencia de diarrela e o desempenho de leitoes recem-d" REVISTA BRASILEIRA DE ZOOTECNIA - BRAZILIAN JOURNAL OF ANIMALSCIENCE, SOCIEDADE BRASILEIRA DE ZOOTECNIA, VICOSA, BR, vol. 35, no. 6, 1 November 2006 (2006-11-01), pages 2359-2367, XP008083385 ISSN: 1516-3598
- BURT S: "ESSENTIAL OILS: THEIR ANTIBACTERIAL PROPERTIES AND POTENTIAL APPLICATIONS IN FOODS-A REVIEW" INTERNATIONAL JOURNAL OF FOOD MICROBIOLOGY, ELSEVIER SCIENCE PUBLISHERS, AMSTERDAM, NL, vol. 94, no. 3, 1 January 2004 (2004-01-01), pages 223-253, XP008056620 ISSN: 0168-1605
- LIPPENS M ET AL: "Effect of the use of coated plant extracts and organic acids as alternativesfor antimicrobial growth promoters on the performance of broiler chickens" ARCHIV FUER GEFLUEGELKUNDE, VERLAG EUGEN ULMER GMBH, DE, vol. 69, no. 6, 1 December 2005 (2005-12-01), pages 261-266, XP008083190 ISSN: 0003-9098
- HERNANDEZ F ET AL: "Influence of two plant extracts on broilers performance, digestibility and digestive organ size" POULTRY SCIENCE, CHAMPAIGN, IL, US, vol. 83, 1 January 2004 (2004-01-01), pages 169-174, XP002450202 ISSN: 0032-5791

## Description

This invention relates to the use of essential oil compounds as defined hereinafter as components of animal feed or drinking water or feed or drinking water additives, as well as to the use of these compounds as histomonastats.

The term animal includes all animals including humans. Examples of animals are non-ruminants, and ruminants. Ruminant animals include, for example, animals such as sheep, goat, and cattle, e.g. cow such as beef cattle and dairy cows. In a particular embodiment, the animal is a non-ruminant animal. Non-ruminant animals include mono-gastric animals, e.g. pig or swine (including, but not limited to, piglets, growing pigs, and sows); poultry such as turkeys, ducks and chickens (including but not limited to broiler chicks, layers); fish (including but not limited to salmon, trout, tilapia, catfish and carp); and crustaceans (including but not limited to shrimp and prawn). In a preferred embodiment, the animals are turkeys, ducks and chickens.

Histomonas is a generic name given to single cell protozoan organisms that are intestinal parasites that infect both vertebrates and invertebrates. Of special interest is the protozoa Histomonas meleagridis. Histomonas meleagridis is a pleomorphic flagellate transmitted in the eggs of the caecal nematode, Heterakis gallinarum. That protozoan cannot exist by itself in the external environment. It is commonly found within gallinaceous birds and may cause a serious disease termed histomoniasis (blackhead). This disease occurs most commonly in turkey poults, although other birds sometimes become diseased. The amoeboid form of the parasite invades the intestinal mucosa, causing ulcerations, peritonitis, and intestinal perforations. Disease is associated with several species of pathogenic bacteria, including Clostridium perfringens and Escherichia coli, and the protozoan appears incapable of causing pathology in the absence of these bacterial pathogens. When pathogenic, the parasite may invade the liver where necrotic lesions form. Infected birds developed ruffled feathers, dark skin pigmentation, and hanging wings and tail.

In order to combat Histomoniasis, the use of mebendazole and related anti-nematode drugs to prevent transmission of the protozoan is known. Additionally animal feeds are often supplemented with a histomonastat. Histomonastats for use with poultry (chickens, turkeys) may include for example 4-nitrophenylarsonic acid, a compound that is non-natural and thus has to be made synthetically. Further it is known that for example the authorized histomonastat for turkeys, Nifursol, was withdrawn in 2003 by the UK authority.

WO 2004/089108 discloses the use of a feed additive containing the essential oil cinnamaldehyde and thiosulfonates for the manufacturing of a composition to prevent or treat animal pathology due H. meleagridis.

WO 2004/091307 is a generic disclosure listing a very large number of essential oils and plant derived bioactives used commercially in the feed food, and flavouring industries. This document further speculates that all these bioactives can be used to treat any type of infectious disease caused by bacteria, fugi, protozoa and viruses in any type of terrestrial and aquatic animal. However, examples are limited to aquatic animals and does not disclose the specific selection of 2-decanal, and nerolidol for prevention and treatment of histomoniasis.

There is therefore a need for new and additional histomonastats that are for example naturally occurring.

The present invention is based on the finding that essential oil compounds selected from the group consisting of 2-decenal and nerolidol have activity against histomoniasis and therefore can be used as histomonastats.

Therefore, this invention relates to the use of an essential oil compound selected from the group hereinabove defined as a histomonastat. Accordingly, the present invention provides a method of treatment, or prophylaxis, of histomoniasis in the human or animal body, the method comprising administering of at least one essential oil compound selected from the group consisting of 2-decenal and nerolidol to that human or animal.

By using an essential oil compound as defined above as a histomonastat, one can employ a naturally occurring compound which is more likely to be acceptable to the animal being treated for histomoniasis. Also, industry and consumer groups are often in favour of using naturally occurring compounds rather than synthetic ones.

A second aspect of the present invention relates to the use of at least one of said essential oil compounds in the preparation of a histomonastatic composition.

A third aspect of the invention relates to an animal feed composition, such as suitable for a monogastric or non-ruminant animal, comprising at least one of an essential oil compound as defined hereinabove which is present as a histomonastat. The essential oil compound or mixture according to the invention is preferably present in an amount at which it has histomonastatic activity.

The term feed or feed composition means any compound, preparation, mixture, or composition suitable for, or intended for intake by an animal.

A fourth aspect of the invention relates to a premix or additive composition, such as to be added to one or more edible feed substance (s) or ingredient (s), for example to prepare or for supplementation to an existing feed to form a feed composition.

In a further aspect, this invention relates to the use of compounds as herein above defined in the manufacture of a pharmaceutical composition for the treatment or prophylaxis of histomoniasis.

In just a further aspect, the present invention relates to a method of treatment, or prophylaxis of histomoniasis in the animal body, the method comprising administering compounds according to the invention to that animal.

The compounds of the invention may be used (i) in therapy, i.e. for treatment of histomonaisis, and/or (ii) for prophylaxis, i.e. treatment to prevent the onset of histomoniasis ("primary" prophylaxis), and/or the recurrence of symptoms in an existing infection that has been brought under control ("secondary" prophylaxis, maintenance therapy).

The compounds of the invention may be used (a) in veterinary medicine, which is the application of medical, diagnostic, and therapeutic principles to companion, domestic, exotic, wildlife, and production animals; and/or (b) in human medicine.

In another aspect, the invention relates to pharmaceutical including veterinary compositions comprising at least one compound of the invention. In a particular embodiment, the present invention relates to a pharmaceutical composition comprising a compound of the invention and a suitable carrier.

The essential oil compound compositions as hereinabove described (i.e. histomonastatic, animal feed composition, premix or additive composition and pharmaceutical composition) may be prepared in accordance with methods known in the art and may be in the form of a liquid or a dry composition. The essential oil compound to be included in the composition may be stabilized in accordance with methods known in the art.

The normal daily dosage of a compound according to the invention provided to an animal by feed intake depends upon the kind of animal and its condition. Normally this dosage should be in the range of from about 5 to about 1000 mg, preferably from about 10 to about 500 mg compound per kg of feed.

In a preferred embodiment of the invention the essential oil compound according to the invention being used in an amount sufficient to provide a daily dosage of 2.5 mg per kg body weight to about 100 mg per kg body weight of the subject to which it is to be administered.

Particular examples of feed compositions of the invention are:
- An animal feed additive comprising (a) at least one of the essential oil compounds of the invention; and (b) at least one fat-soluble vitamin, (c) at least one water-soluble vitamin, (d) at least one trace mineral, and/or (e) at least one macro mineral;
- an animal feed composition having a crude protein content of 50 to 800 g/kg and comprising at least one of the essential oil compounds of the invention.

The so-called premixes are examples of animal feed additives of the invention. A premix designates a preferably uniform mixture of one or more micro-ingredients with diluent and/or carrier. Premixes are used to facilitate uniform dispersion of micro-ingredients in a larger mix.

In the use according to the invention the essential oil compound can be fed to the animal before, after, or simultaneously with the diet. The latter is preferred.

In a particular embodiment, the essential oil compound, in the form in which it is added to the feed, or when being included in a feed additive, is well-defined. The term well-defined means that the essential oil compound preparation which contains the compound and which is used to be added to the feed or feed additive is at least 30% pure.

A well-defined essential oil compound preparation is advantageous. For instance, it is much easier to dose correctly to the feed an essential oil compound that is essentially free from interfering or contaminating other compounds. The term dose correctly refers in particular to the objective of obtaining consistent and constant results, and the capability of optimising dosage based upon the desired effect.

For the use in animal feed, however, the essential oil compound need not be that pure; it may e.g. include other essential oil compounds according to the invention or other active compounds which improve the general health status of the animal such as oregano oil, in which case it could be termed an essential oil compound preparation.

The use of mixtures of the three essential oil components is preferred. According to the invention the compound preparation may additionally contain other essential oil compounds as for example p-cymene, thymol or for example salicylaldehyde as exemplified in example 6 hereinafter or tea tree oil, peppermint oil, cuminaldehyde, cinnamic acid, cinnamic alcohol, farnesal or farnesylacetone.

The essential oil compound preparation can be (a) added directly to the feed (or used directly in a treatment process of proteins), or (b) it can be used in the production of one or more intermediate compositions such as feed additives or premixes that is subsequently added to the feed (or used in a treatment process). The degree of purity described above refers to the purity of the original essential oil compound preparation, whether used according to (a) or (b) above.

A part from the essential oil compound of the invention, animal feed additives of the invention contain at least one fat-soluble vitamin, and/or at least one water soluble vitamin, and/or at least one trace mineral, and/or at least one macro mineral.

Further, optional, feed-additive ingredients are colouring agents, e.g. carotenoids such as beta-carotene, canthaxanthin, apoester, astaxanthin, and lutein; aroma compounds; stabilisers; coccidiostats, antimicrobial peptides; polyunsaturated fatty acids (PUFAs); reactive oxygen generating species; and/or at least one enzyme selected from amongst phytase (EC 3.1.3.8 or 3.1.3.26); xylanase (EC 3.2.1.8); galactanase (EC 3.2.1.89); alpha-galactosidase (EC 3.2.1.22); protease (EC 3.4., phospholipase A1 (EC 3.1.1.32); phospholipase A2 (EC 3.1.1.4); lysophospholipase (EC 3.1.1.5); phospholipase C (EC 3.1.4.3); phospholipase D (EC 3.1.4.4); amylase such as, for example, alpha-amylase (EC 3.2.1.1); and/or beta-glucanase (EC 3.2.1.4 or EC 3.2.1.6).

Examples of antimicrobial peptides (AMP's) are CAP18, Leucocin A, Protegrin-1, Thanatin, Defensin, Lactoferrin, Lactoferricin, and Ovispirin such as Novispirin (Robert Lehrer, 2000), Plectasins, and Statins, including the compounds and essential oil compounds disclosed in WO 03/044049 and WO 03/048148, as well as variants or fragments of the above that retain antimicrobial activity.

Examples of polyunsaturated fatty acids are C18, C20 and C22 polyunsaturated fatty acids, such as arachidonic acid, docosohexaenoic acid, eicosapentaenoic acid and gamma-linoleic acid.

Examples of reactive oxygen generating species are chemicals such as perborate, persulphate, or percarbonate; and enzymes such as an oxidase, an oxygenase or a syntethase.

Usally fat- and water-soluble vitamins, as well as trace minerals form part of a so-called premix intended for addition to the feed, whereas macro minerals are usually separately added to the feed. Either of these composition types, when enriched with a essential oil compound is an animal feed additive of the invention.

The following are non-exclusive lists of examples of these components:
- Examples of fat-soluble vitamins are vitamin A, vitamin D3, vitamin E, and vitamin K, e.g. vitamin K3.
- Examples of water-soluble vitamins are vitamin C, vitamin B 12, biotin and choline, vitamin B1, vitamin B2, vitamin B6, niacin, folic acid and panthothenate, e.g. Ca-D-panthothenate.
- Examples of trace minerals are manganese, zinc, iron, copper, iodine, selenium, and cobalt.
- Examples of macro minerals are calcium, phosphorus and sodium.

The nutritional requirements of these components (exemplified with poultry and piglets/pigs) are listed in Table A of WO 01/58275. Nutritional requirement means that these components should be provided in the diet in the concentrations indicated.

Animal feed compositions or diets have a relatively high content of protein. Poultry and pig diets can be characterised as indicated in Table B of WO 01/58275, columns 2-3. Fish diets can be characterised as indicated in column 4 of this Table B. Furthermore such fish diets usually have a crude fat content of 200-310 g/kg.

WO 01/58275 corresponds to US 09/779334

An animal feed composition according to the invention has a crude protein content of 50-800 g/kg, and furthermore comprises at least one essential oil compound as described and/or claimed herein.

In a particular embodiment, the animal feed composition of the invention contains at least one vegetable protein or protein source. It may also contain animal protein, such as Meat and Bone Meal, and/or Fish Meal, typically in an amount of 0-25%. The term vegetable proteins as used herein refers to any compound, composition, preparation or mixture that includes at least one protein derived from or originating from a vegetable, including modified proteins and protein-derivatives. In particular embodiments, the protein content of the vegetable proteins is at least 10, 20, 30, 40, 50, or 60% (w/w).

Vegetable proteins may be derived from vegetable protein sources, such as legumes and cereals, for example materials from plants of the families Fabaceae (Leguminosae), Cruciferaceae, Chenopodiaceae, and Poaceae, such as soy bean meal, lupin meal and rapeseed meal. In a particular embodiment, the vegetable protein source is material from one or more plants of the family Fabaceae, e.g. soybean, lupine, pea, or bean. In another particular embodiment, the vegetable protein source is material from one or more plants of the family Chenopodiaceae, e.g. beet, sugar beet, spinach or quinoa.

Animal diets can e.g. be manufactured as mash feed (non pelleted) or pelleted feed. Typically, the milled feed-stuffs are mixed and sufficient amounts of essential vitamins and minerals are added according to the specifications for the species in question. The essential oil compound(s) can be added as solid or liquid formulations. For example, a solid essential oil compound formulation is typically added before or during the mixing step; and a liquid essential oil compound preparation is typically added after the pelleting step. The essential oil compound may also be incorporated in a feed additive or premix.

It is at present contemplated that the essential oil compound is administered in one or more of the following amounts (dosage ranges): 0.5-1000; 1-500; 50-250; all these ranges being in mg essential oil compound(s) according to the invention per kg feed (ppm). Examples of particularly preferred dosages are 25, 50, 100, 125, 250 and 500 ppm.

As mentioned hereinabove, the invention also relates to the pharmaceutical use of the essential oil compounds , 2-decenal and nerolidol. Accordingly, this aspect of the invention includes:
I. Use of said essential oil compounds in the preparation of a pharmaceutical formulation, e.g. medicament, for the treatment or prophylaxis of histomoniasis.
II. A method of medical treatment comprising administering an essential oil compound according to the invention to an individual in need of medical treatment against histomoniasis.
III. A pharmaceutical composition comprising at least one of said essential oil compounds and a suitable carrier for use as a histomonastat.

A pharmaceutical composition according to the invention may be prepared in accordance with methods known in the art and may be in the form of a liquid or a dry composition. The essential oil compound according to the invention to be included in the composition may be stabilized in accordance with methods known in the art.

The dosage of the essential oil compound composition of the invention and other conditions under which the composition is used may be determined on the basis of methods known in the art.

Generally, the composition of the invention comprises an effective amount of at least one essential oil compound of the invention. The term "effective amount" when used herein is intended to mean an amount of the essential oil compounds of the invention, which is sufficient to inhibit growth of the protozoa in question.

Formulations of the essential oil compounds of the invention may be administered to a host suffering from or predisposed to a histomonas infection. Generally the dose of the essential oil compound or compounds of the invention will be sufficient to decrease the protozoa population by at least about 50%. The essential oil compounds (or compounds) of the present invention may be administered at a dosage that reduces the protozoa population while minimizing any side-effects. It is contemplated that the composition will be obtained and used under the guidance of a physician or veterinarian for in vivo use.

Various methods for administration may be employed. The pharmaceutical formulation may be given orally, or may be injected intravascularly, subcutaneously, peritoneally, by aerosol, opthalmically, intra-bladder, topically, etc. The dosage of the therapeutic formulation will vary widely, depending on the frequency of administration, the manner of administration and the like. The initial dose may be larger, followed by smaller maintenance doses. The dose may be administered as infrequently as weekly or biweekly, or fractionated into smaller doses and administered once or several times daily, semi-weekly, etc. to maintain an effective dosage level. In many cases, oral administration will require a higher dose than if administered intravenously. Examples of particularly preferred dosage ranges are: 500 - 1000, 1000-1500 and 1500 to 2000 ppm / day.

The compounds of this invention can be incorporated into a variety of formulations for therapeutic administration. More particularly, the compounds of the present invention can be formulated into pharmaceutical compositions by combination with appropriate, pharmaceutically acceptable carriers or diluents, and may be formulated into preparations in solid, semi-solid, liquid or gaseous forms, such as tablets, capsules, powders, granules, ointments, creams, foams, solutions, suppositories and injections. As such, administration of the compounds can be achieved in various ways, including oral, buccal, rectal, parenteral, intraperitoneal, intradermal, transdermal, intracheal, etc., administration. The essential oil compounds of the invention may be localized by the use of an implant or other formulation that acts to retain the active dose at the site of implantation.

For oral preparations, the compounds may be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

The compounds may be formulated into preparations for injections by dissolving, suspending or emulsifying them in an aqueous or non-aqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

Unit dosage forms for oral or rectal administration such as syrups, elixirs, and suspensions may be provided wherein each dosage unit, for example, teaspoonful, tablespoonful, tablet or suppository, contains a predetermined amount of the composition containing one or more compounds of the present invention. Similarly, unit dosage forms for injection or intravenous administration may comprise the compound of the present invention in a composition as a solution in sterile water, normal saline or another pharmaceutically acceptable carrier.

Implants for sustained release formulations are well-known in the art. Implants are formulated as microspheres, slabs, etc. with biodegradable or non-biodegradable polymers. For example, polymers of lactic acid and/or glycolic acid form an erodible polymer that is well-tolerated by the host. The implant containing the antimicrobial essential oil compounds of the invention is placed in proximity to the site of infection, so that the local concentration of active agent is increased relative to the rest of the body.

The term "unit dosage form", as used herein, refers to physically discrete units suitable as unitary dosages for human and animal subjects, each unit containing a predetermined quantity of compounds of the present invention calculated in an amount sufficient to produce the desired effect in association with a pharmaceutically acceptable diluent, carrier or vehicle. The specifications for the unit dosage forms of the present invention depend on the particular compound employed and the effect to be achieved, and the pharmacodynamics associated with the compound in the host.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

The composition may further comprise another pharmaceutically active agent, such as an antimicrobial compound exhibiting antimicrobial activity. The antimicrobial compound may be an antibiotic, as known in the art. Classes of antibiotics include penicillins, e.g. penicillin G, penicillin V, methicillin, oxacillin, carbenicillin, nafcillin, ampicillin, etc.; penicillins in combination with beta-lactamase inhibitors, cephalosporins, e.g. cefaclor, cefazolin, cefuroxime, moxalactam, etc.; carbapenems; monobactams; aminoglycosides; tetracyclines; macrolides; lincomycins; polymyxins; sulfonamides; quinolones; cloramphenical; metronidazole; spectinomycin; trimethoprim; vancomycin; etc. The biocidal agent may also be an anti-mycotic agent, including polyenes, e.g. amphotericin B, nystatin; 5-flucosyn; and azoles, e.g. miconazol, ketoconazol, itraconazol and fluconazol.

The invention described and claimed herein is not to be limited in scope by the specific embodiments herein disclosed, since these embodiments are intended as illustrations of several aspects of the invention. Indeed, various modifications of the invention in addition to those shown and described herein will become apparent to those skilled in the art from the foregoing description.

The following examples further illustrate the invention.

### Example 1: Animal Feed Additive

An animal feed additive is prepared by adding 10 g of at least one of the essential oil compounds according to the invention to the following premix (per kilo of premix):

| | |
|---|---|
| 1100000 IE | Vitamin A |
| 300000 IE | Vitamin D3 |
| 4000 IE | Vitamin E |
| 250 mg | Vitamin B1 |
| 800 mg | Vitamin B2 |
| 1200 mg | Ca-D-Panthothenate |
| 500 mg | Vitamin B6 |
| 2.5 mg | Vitamin B 12 |
| 5000 mg | Niacin |
| 10000 mg | Vitamin C |
| 300 mg | Vitamin K3 |
| 15 mg | Biotin |
| 150 mg | Folic acid |
| 50004 mg | Cholin chloride |
| 6000 mg | Fe |
| 3000 mg | Cu |
| 5400 mg | Zn |
| 8000 mg | Mn |
| 124 mg | I |
| 60 mg | Co |
| 29.7 mg | Se |
| 9000 mg | Lasalocid Sodium (Avatec) |
| 17.3 % | Ca |
| 0.8 % | Mg |
| 11.7 % | Na |

### Example 2: Animal Feed

A broiler grower diet having the following composition (%, w/w) is prepared by mixing the ingredients. Wheat, rye and SBM 48 are available from Moulin Moderne Hirsinque, Hirsingue, France. After mixing, the feed is pelleted at a desired temperature, e.g. about 70°C (3 x 25 mm).

| | |
|---|---|
| Wheat | 46.00 |
| Rye | 15.00 |
| Soy Bean Meal (SBM 48) | 30.73 |
| Soybean oil | 4.90 |
| DL-Methionine | 0.04 |
| DCP (Di-Calcium Phosphate) | 1.65 |
| Limestone | 0.43 |
| Salt | 0.15 |
| TiO2 | 0.10 |
| Animal feed additive (above) | 1.00 |

The resulting animal feed comprises 100 mg essential oil compound according to the invention per kg (100 ppm).

### Example 3:

A broiler chicken feed ("starter") containing at least one of a essential oil compound according to the invention selected from Tab. 1 can be prepared by mixing the following ingredients together using a conventional mixing apparatus at room temperature.

| Ingredient | Amount (kg) |
|---|---|
| Soybean meal | 34.50 |
| Maize | 20.00 |
| Wheat | 37.80 |
| Soy oil | 3.13 |
| Minerals | 2.90 |
| Synthetic amino acids premix | 0.17 |
| Vitamins and trace elements premix | 1.00 |
| essential oil compound | 5.00 g |

### Example 4:

A broiler chicken food ("grower") containing at least one of a essential oil compound according to the invention selected from Tab. 1 can be prepared by mixing the following ingredients together using a conventional mixing apparatus at room temperature.

| Ingredients | Amount (kg) |
|---|---|
| Soybean meal | 31.2 |
| Maize | 20.0 |
| Wheat | 41.3 |
| Soy oil | 3.4 |
| Minerals | 2.5 |
| Synthetic amino acids premix | 0.1 |
| Vitamins and trace elements premix | 1.0 |
| essential oil compound | 5.0 g |

In principle the essential oil compound according to the invention premix may contain 2 - 10% of the essential oil compound according to the invention derivative.

### Example 5: The antimicrobial activity of the compounds of the invention towards Histomonas meleagridis

The in vitro activity of the several essential oil compounds was assessed using the following technique. Aliquots of 1 ml of H. meleagridis culture (10⁶ parasites/ml) were incubated anaerobicaly for 24 h at 39°C. Twelve two-fold dilutions of a stock solution of each compound were prepared in 96-well U bottom plates (Prolabo, Lyon, France) in Tween 80 (Sigma, L'Isle d'Abeau, France) and added to the parasites. The initial dilution was 2% (2000 ppm). After incubation for 24 h and 48 h, the number of live parasite in each culture was estimated by vital coloration with Trypan blue 0.4% (Gibco, BRL Life technology, Cergy Pontoise, France). The lowest dilution in which no live organisms were observed was defined as the minimal lethal concentration (MLC).

The results are shown in Table 1.

**Table 1: Lethal activity against histomonas in ppm (of active compound)**

| **Compound / ppm** | **1000** | **500** | **250** | **125** |
|---|---|---|---|---|
| cinnamaldehyde, trans- | X | X | X | |
| cymene, p- | X | X | | |
| thymol | X | X | | |
| tea tree oil | X | | | |
| peppermint oil | X | | | |
| cuminaldehyde | X | | | |
| cinnamic acid | X | | | |
| cinnamic alcohol | X | | | |
| decenal, 2- | X | X | X | |
| salicylaldehyde | X | X | | |
| nerolidol | X | X | X | X |
| farnesal | X | X | | |
| farnesylacetone | X | | | |

The MLC for cinnamaldehyde and 2-decenal are 250 ppm and for nerolidol the MLC is 125 ppm. For p-cymene and salycilaldehyde the MLC are 500 ppm.

## Claims

1. 2-decenal and/or nerolidol for use in the treatment or prophylaxis of histomoniasis.

2. An animal feed composition or an additive or a premix composition thereof, comprising 2-decenal and/or nerolidol.

3. An animal feed composition or an additive or a premix composition according to claim 2 which further comprises at least one compound selected from the group consisting of p-cymene, thymol, salicylaldehyde, tea tree oil, peppermint oil, cuminaldehyde, cinnamic acid, cinnamic alcohol, farnesal and farnesylacetone.

4. An animal feed additive according to any of claims 2 to 3 comprising
(a) at least one fat-soluble vitamin,
(b) at least one water-soluble vitamin,
(c) at least one trace mineral, and/or
(d) at least one macro mineral.

## Patentansprüche

1. 2-Decenal und/oder Nerolidol für die Verwendung bei der Behandlung oder Prophylaxe von Histomoniasis.

2. Tierfuttermittelzusammensetzung oder ein Zusatzstoff oder eine Vormischungszusammensetzung davon, umfassend 2-Decenal und/oder Nerolidol.

3. Tierfuttermittelzusammensetzung oder ein Zusatzstoff oder eine Vormischungszusammensetzung nach Anspruch 2, die/der weiterhin mindestens eine Verbindung, ausgewählt aus der Gruppe bestehend aus p-Cymol, Thymol, Salicylaldehyd, Teebaumöl, Pfefferminzöl, Kuminaldehyd, Zimtsäure, Zimtalkohol, Farnesal und Farnesylaceton, umfasst.

4. Tierfuttermittelzusatzstoff nach einem der Ansprüche 2 bis 3, umfassend
(a) mindestens ein fettlösliches Vitamin,
(b) mindestens ein wasserlösliches Vitamin,
(c) mindestens einen mineralischen Mikronährstoff und/oder
(d) mindestens einen mineralischen Makronährstoff.

## Revendications

1. 2-Décénal et/ou nérolidol destinés à être utilisés dans le traitement ou la prophylaxie de l'histomonose.

2. Composition d'aliment pour animaux ou additif ou composition de prémélange de celui-ci, comprenant du 2-décénal et/ou du nérolidol.

3. Composition d'aliment pour animaux ou additif ou composition de prémélange selon la revendication 2, qui comprend en outre au moins un composé choisi dans le groupe constitué par le p-cymène, le thymol, le salicylaldéhyde, l'essence d'arbre à thé, l'essence de menthe poivré, le cuminaldéhyde, l'acide cinnamique, l'alcool cinnamique, le farnésal et la farnésylacétone.

4. Additif d'aliment pour animaux selon l'une ou l'autre des revendications 2 et 3, comprenant
(a) au moins une vitamine lyposoluble,
(b) au moins une vitamine hydrosoluble,
(c) au moins un oligo-minéral, et/ou
(d) au moins un macro-minéral.
